# EUROPEAN PATENT SPECIFICATION

(11) **EP 3 937 879 B1**
(45) Date of publication and mention of the grant of the patent: **29.04.2026**
(21) Application number: 20769098.3
(22) Date of filing: 16.03.2020
(51) Int. Cl.: A61K 31/7024, A61K 31/235, A61K 33/16, A61K 33/30, A61K 36/48, A61K 6/00, A61K 8/19, A61K 8/25, A61P 31/14, A61K 8/27

(54) **PREPARATIONS FOR THE PREVENTION OF CORONAVIRUS INFECTIONS ACQUIRED VIA THE ORAL CAVITY AND PHARYNX**
ZUBEREITUNGEN ZUR VORBEUGUNG VON CORONOVARIUSINFEKTIONEN, DIE ÜBER DIE MUNDHÖHLE UND DEN RACHENRAUM ERWORBEN WERDEN
PRÉPARATION POUR LA PRÉVENTION DE INFECTIONS PAR CORONAVIRUS ACQUISES PAR L'INTERMÉDIAIRE DE LA CAVITÉ BUCCALE ET DU PHARYNX

(30) Priority: 14.03.2019 US 201916353802
(43) Date of publication of application: 19.01.2022
(73) Proprietor: MangoRx IP Holdings, LLC, Dallas, TX 75248 (US)
(72) Inventor: MONTES, Joseph, G., New Orleans, LA 70127 (US); INTRATER, James, Hackensack, New Jersey 07601 (US)
(74) Representative: Pearl Cohen UK
(86) International application number: PCT/US2020/022903
(87) International publication number: WO 2020/186258

(56) References cited:
- WO-A1-2016/067283
- WO-A1-2016/067283
- WO-A1-2019/055312
- WO-A2-2004/098566
- WO-A2-2013/071288
- US-A- 4 146 606
- US-A1- 2015 182 445
- US-A1- 2015 224 202
- US-A1- 2019 209 470
- DATABASE GNPD [online] MINTEL; 17 March 2017 (2017-03-17), ANONYMOUS: "Thera Zinc Spray", XP055800381, retrieved from https://www.gnpd.com/sinatra/recordpage/4688265/ Database accession no. 4688265
- AGUILAR-GALVEZ ANA ET AL: "Potential of tara (Caesalpinia spinosa)gallotannins and hydrolysates as natural antibacterial compounds", FOOD CHEMISTRY, ELSEVIER LTD, NL, vol. 156, 6 February 2014 (2014-02-06), pages 301 - 304, XP028632758, ISSN: 0308-8146, DOI: 10.1016/J.FOODCHEM.2014.01.110
- CHUNG K-T ET AL: "Tannins and Human Health: A Review", CRITICAL REVIEWS IN FOOD SCIENCE AND NUTRITION, TAYLOR & FRANCIS, USA, vol. 38, no. 6, 1 August 1998 (1998-08-01), pages 421 - 464, XP002979210, ISSN: 1040-8398, DOI: 10.1080/10408699891274273
- RAO ET AL.: "Zinc for the common cold— not if, but when", ZINC FOR THE COMMON COLD-NOT IF, BUT WHEN, vol. 60, no. 11, November 2011 (2011-11-01), pages 669 - 671, XP055739113, Retrieved from the Internet <URL:https://www.ncbi.nlm.nih.gov/pmc/articles/PMC3273967/pdf/JFP-60-669.pdf> [retrieved on 20200522]
- ANONYMOUS: "Tannic Acid", WIKIPEDIA, THE FREE ENCYCLOPEDIA, 2 February 2019 (2019-02-02), XP055739116, Retrieved from the Internet <URL:https://en.wikipedia.org/wiki/Tannic_acid> [retrieved on 20200526]

## Description

### FIELD OF THE INVENTION

The invention relates to compositions and formulations useful for preventing or inhibiting the acquisition of infections via the oral cavity, pharynx, and/or lungs. Specifically, the invention relates to toothpastes, in the form of a paste or gel which are useful in the prevention or inhibition of upper wots respiratory and pharyngeal coronavirus infections.

### BACKGROUND OF THE INVENTION

A number of toothpastes and mouthwashes already on the market are known to fight tooth decay (usually through fluoride), to reduce the incidence of gingivitis (usually by the action of triclosan and/or of a zinc salt, such as zinc acetate, zinc gluconate, zinc lactate, or zinc chloride (M. Sanz, et al., Antiplaque and Antigingivitis toothpastes. Monograph Oral Sci (2013) 23:27-44)), to help clean out the mouth of food particles, and to produce "fresh breath." Thus, while currently available over-the-counter toothpastes address the need for the prevention of dental caries (bacteria-induced tooth decay leading to cavities), gingivitis, amelioration of sensitive teeth, and oral hygiene in general, there is a need in the art for toothpastes and other orally administered preparations that includes ingredients that will enhance the ability of the toothpaste or preparation to prevent or inhibit the acquisition of or reduce the incidence or likelihood of a number of conditions, including the common cold and a host of other pathological conditions caused by pathogenic viruses, bacteria, and fungi, or possibly other conditions (e.g., canker sores, ear infections, etc.). Thus, there is a need in the art for toothpastes and mouthwashes and other formulations that will prevent or inhibit the acquisition of or reduce the incidence or likelihood of a number of infections via the oral cavity and the pharynx, while at the same time function as most toothpastes and mouthwashes in preventing cavities and cleansing the oral cavity.

WO 2019/055312 discloses a toothpaste or other orally applied product to be used at least once daily to help prevent or inhibit the acquisition of a number of infections by oral or pharyngeal tissues WO 2013/071288 A2 discloses the use of zinc gluconate, e.g. by buccal or nasopharyngeal administration, for the prevention and inhibition of bacterial, bacterial, fungal infections, including influenza, coronavirus, SARS, and HCV.

### SUMMARY OF THE INVENTION

Described here are formulations that when applied to the mouth, pharynx or lung in the form of one of its embodiments will help prevent or inhibit the acquisition of a large number of possible pathogenic conditions that originate from interaction between a virus and tissues present in the mouth, pharynx and/or lungs.

The present invention provides a composition formulated as a toothpaste for use in the prevention or inhibition of upper respiratory and pharyngeal infections, wherein the infection is a coronavirus infection, the composition comprising a dentifrice gel or paste comprising: (1) a free zinc salt, wherein the free zinc salt ranges from 0.1 % to 0.5 % by weight of the composition; and (2) a highly purified extract of gallic tannins or tara tannins that do not stain tooth enamel, and wherein the tannins are 0.01 % to 10 % by weight of the composition.

In a preferred embodiment, the compositions and formulations described herein can be delivered adequately into the oral and pharyngeal surfaces. By the customary--at least dailybrushing of the teeth, followed by minimal rinsing or merely spitting out of the preparation the user will be protected from acquiring or have a lower incidence of a number of pathogenic conditions.

As a result, the user will be protected from acquiring or have a lower incidence of a number of viral infections, such as SARS or COVID-19.

In an embodiment, the composition is stored in a malleable or squeezable tube.

In an embodiment, the coronavirus infection is COVID-19.

In an embodiment, the zinc salt is an organic zinc salt selected from zinc gluconate, zinc lactate, zinc acetate, or combinations thereof.

In an embodiment, the zinc salt is an inorganic zinc salt selected from zinc chloride, zinc sulphate, zinc carbonate, or combinations thereof.

In an embodiment, the composition further comprises a fluoride compound.

In an embodiment, the tara tannins are a highly purified extract from pods from *Caesalpinia spinosa.*

In an embodiment, the dentifrice gel or paste comprises a colorless or low-color tannin (including GALALCOOL^{®}, a low-color tannin extracted from tara pods for addition to white wine and sold by Laffort Company based in France, and tara tannins, or another mass-produced, colorless or low-color or white-colored tannin, or a combination thereof). In some embodiments, the toothpaste or other orally administered preparation further comprises zinc protoporphyrin IX.

Other features and advantages of the present invention will become apparent from the following detailed description examples. It should be understood, however, that the detailed description and the specific examples, while indicating preferred embodiments of the invention, are given by way of illustration only. The present invention is defined by the claims. In particular, the prevention of infections mentioned in the present description other than those caused by coronavirus, are not part of the present invention.

### DETAILED DESCRIPTION OF THE INVENTION

**In** the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The present invention provides a composition formulated as a toothpaste for use in the prevention or inhibition of upper respiratory and pharyngeal infections, wherein the infection is a coronavirus infection, the composition comprising a dentifrice gel or paste comprising: (1) a free zinc salt, wherein the free zinc salt ranges from 0.1 % to 0.5 % by weight of the composition; and (2) a highly purified extract of gallic tannins or tara tannins that do not stain tooth enamel, and wherein the tannins are 0.01 % to 10 % by weight of the composition. In some embodiments, the toothpaste contains a base preparation with suitable ingredients known in the art, such as a fluoride, a detergent (such as sodium lauryl sulfate), a solvent (such as glycerol and/or propylene glycol), an abrasive (such as fumed silica), an antibacterial (such as triclosan), a flavoring agent, including a sweetener. In an embodiment, one or more of the following is added to the base preparation or formulation in addition to the highly purified extract of gallic tannins or tara tannins:
(1) an astringent compound, a porphyrin, called zinc protoporphyrin IX; and
(2) an astringent compound, a colorless or low-color tannin other than GALALCOOL^{®} or tara tannins (e.g., TANFRESH^{®}, a low color tannin preparation of proanthocyanidic tannins and oak ellagic tannins, from the same company as GALALCOOL^{®})

The above ingredients will produce their effects on preventing or inhibiting the acquisition of a number of pathogens, including viruses that cause colds, such as rhinovirus, including a number of oral bacteria that may be pathogenic, for example, those that may result in gingivitis, and including oral fungi that may produce thrush. Typically, the user when employing a toothbrush to apply a toothpaste described herein will brush with the toothpaste at least once a day, and immediately following brushing will either non-vigorously spit out the preparation only once and/or rinse it out with water.

The toothpaste will prevent or inhibit the acquisition of or reduce the incidence or likelihood of a number of infections via the oral cavity and the pharynx, while at the same time function as most toothpastes and mouthwashes in preventing cavities and cleansing the oral cavity.

One of the present inventors was commonly beset with the common cold most of his life, typically acquiring 3 to 4 colds per year. However, he then started to correlate lower incidence of his own colds to select ingredients a person might place deliberately in his mouth. Some have been advanced a probable scientific basis, while others have been anecdotally linked to lower incidence of disease, including:
1. Salts of zinc;
2. Stannous fluoride or other fluoride;
3. Sodium lauryl sulfate; and
4. Polyphenols, such as tannins.

A number of toothpastes and mouthwashes already on the market are known to fight tooth decay (usually through fluoride), to reduce the incidence of gingivitis (usually by the action of triclosan and/or of a zinc salt, such as zinc acetate, zinc gluconate, zinc lactate, or zinc chloride (M. Sanz, et al., Antiplaque and Antigingivitis toothpastes. Monograph Oral Sci (2013) 23:27-44)), to help clean out the mouth of food particles, and to produce "fresh breath." However, no toothpaste or mouthwash has the unique combination of ingredients of the present invention; in particular, an ingredient in the latter toothpaste provides for the slower release of zinc salts. Zinc salts have been used for many years in a number of toothpastes to prevent gingivitis, but given that zinc salts can also be used to reduce the symptoms of the common cold by apparently reducing the infectivity of the cold virus to mucous membranes, it makes sense to provide for a steadier source of zinc ions by using a composition, described below, for rendering zinc salts present in the oral cavity for a longer time period than normally achieved by brushing with current toothpastes. The new toothpaste is typically used at least once per day through brushing with a toothbrush; nonetheless, it can also be used two or more times per day without ill effects or reduction in efficacy. After brushing, the user is to spit out the ingredients from his mouth by no more than one rinse with water, or even without partially or fully rinsing, in order to allow for the adequate retention of residues of the toothpaste.

With regards to the zinc salts, it is now known that zinc ions can prevent the attachment of rhinovirus to cells, and that zinc-containing lozenges can mitigate the symptoms and duration of the common cold (*See* WedMD. Cold, Flu & Cough Health Center - Zinc for Colds: Lozenges & Nasal Sprays. Available at www.webmd.com/cold-and-flu/cold-guide/zinc-lozenges-cold-remedy; and National Institutes of Health Office of Dietary Supplements. Zinc Fact Sheet for Health Professionals. Available at ods.od.nih.gov/factsheets/Zinc-HealthProfessional). However, the observation that one-and-a-half days of not exposing himself to such ingredients resulted in again the acquiring a common cold by one of the inventors, suggested that extension of the residing time of the anti-colds ingredients in the mouth and pharynx could enhance the effectiveness of cold prevention by zinc compounds. Thus, one of the inventors came up with a composition for accomplishing the latter goal of the toothpaste. It should be noted that zinc seems to have effects on other common viruses, such as respiratory syncytial virus (Effect of Zinc Salts on Respiratory Syncytial Virus Replication - NCBI. Available at www.ncbi.nlm.nih.gov/pmc/articles/PMC353050), and thus extending the residing time of zinc salts could produce other health benefits of the toothpaste.

There may be a host of infectious viruses whose ability to infect individuals can be hampered by a toothpaste that contains zinc and applies the latter to the oral cavity, gums, and pharynx. In addition, zinc salts are known to have anti-bacterial and anti-fungal properties as well, that along with fluoride salts, detergents, and other components will also help reduce the frequency of acquiring fungal and bacterial infections. Thus, embodiments of the invention address a broad spectrum of pathologies acquired local to the oral cavity and the pharynx.

A property of zinc salts that correlates with their ability to prevent acquisition of colds (rhinoviruses and other viruses) is that of astringency (*see* www.britannica.com/science/astringent. Accessed 9/14/2016). Zinc belongs in the class of astringents called "metallic astringents", namely those that cause coagulation effects on the surface layers of cells. This would suggest that coagulation of proteins is involved in prevention or inhibition of acquisition of colds. Given that protein coats called capsids surround all viruses, coagulation of the proteins in the coats would radically interfere with the attachment of the virions (individual virus particles) to their cell hosts; this would apply specifically to the host cells superficially exposed to astringents at their surfaces. However, many viruses also have an "envelope" of lipid surrounding the protein coat, so that coagulation effects of an astringent may be interfered with; furthermore, such an envelope is believed to interfere with immune defenses by interfering with protein (virus)-to-protein (antibody) binding. However, toothpastes and other oral and pharyngeal preparations contain surfactants of high efficiency, such as sodium lauryl sulfate, thus helping to solubilize the lipid envelopes of the viruses and thus resulting in the exposure of the protein capsid to the astringent in the preparation. Thus, it appears that a detergent environment is a co-factor in the successful prevention by an astringent (including a zinc salt) of the acquisition of an infection from an enveloped virus. As most colds are caused by rhinoviruses, and they are not enveloped viruses, the need for a detergent co-factor to prevent acquiring them in the pharynx is probably not significant.

In some embodiments of the invention, the coronavirus infection is a *Severe acute respiratory syndrome-related coronavirus* (members of which are responsible for Severe acute respiratory syndrome (SARS) and for COVID-19) (medimoon.com/2014/03/list-of-some-common-viral-diseases-and-their-treatment). It should be noted that the point of initial acquisition of virus is not necessarily representative of the virus's final distribution and symptomatology. For example, one may acquire hepatitis C through the pharynx, but the primary focus of the disease is the liver. By the same token, an inner ear infection (otitis media) can be secondarily acquired from a viral or bacterial infection acquired first in the pharynx. The toothpastes for use according to the present invention prevent or inhibit the acquisition of or reduce the incidence or likelihood of coronavirus infections and in particular COVID-19.

Thus, the property of astringency is linked to stopping the acquisition of viruses by cells of mucous membranes. In embodiments of the present invention, an isolated tannin called GALALCOOL^{®} is an ingredient that will be used to prevent the acquisition of pathological conditions caused by microbes, including viruses and bacteria, via the oral cavity and pharynx. This is at least in part because of its known astringent properties. It will act as a coagulation agent as in the case of zinc. However, unlike zinc alone, it will have an affinity for mucus secretions not expected with the ionic form of zinc (from zinc salts). Thus, it may be able to adhere for longer times to certain mouth and pharyngeal surfaces. Furthermore, it has a beneficial anti-inflammatory property as indicated in at least one study (P. Buzzini, et al., Antimicrobial and Antiviral Activity of Hydrolysable Tannins. Mini-Reviews in Medicinal Chemistry (2008) 8:1179-1187; Yuan-Jin Guo et al., Effect of Corilagin on Anti-inflammation in HSV-1 Encephalitis and HSV-1 Infected Microglias. European Journal of Pharmacology (2010) 635:79-86; J. Luoqi, et al., A Potential Anti-tumor Herbal Medicine, Corilagin, Inhibits Ovarian Cancer Cell Growth Through Blocking the TGF-β Signaling Pathways. BMC Complementary and Alternative Medicine (2013) 13:33; N. Bismelah, et al., Journal of Medicinal Plants Studies (2016) 4:18-23; A Gupta, et al., Phytochemistry and Pharmacological Activities of Haritaki-A Review. Journal of Pharmacy Research (2010) 3:417-424). While an extract of the Indian gooseberry has been proposed as a toothpaste ingredient (P Potduang, et al., The Development of Phyllanthus emblica and Zanthoxylum limonella Toothpaste. The 6th International Conference on Natural Products for Health and Beauty (NATPRO6), January 21-23, 2016), and that extract has a tannin, corilagin as one of its constituents, many other constituents exist in that extract. Furthermore, in that case, corilagin was not singled out as the active ingredient in the preparation nor was its mode of action as an antimicrobial and anti-inflammatory revealed. It should be noted also that the preferred concentration of GALALCOOL^{®}, tara tannins, or other low-color or colorless tannin to be used in embodiments of the present invention is 0.03% by weight, which is much lower than the extract concentration maximum of 0.2% by weight in the toothpaste with the extract proposed by P Potduang, *et al.* However, a much higher concentration of a tannin may prove optimal, and as per claims of this patent, it may be as high as 10% of the toothpaste composition by weight, which is very much higher than 0.2%. In such a case, the concentration of pure tannin in embodiments of the present invention will exceed that in the Indian gooseberry toothpaste by many factors.

Other astringent compounds, namely tannins that are colorless or of low color, would be alternative ingredients to GALALCOOL^{®} or tara tannins, although one or more such tannins may also be used together with the latter to extend efficacy of the dentifrice. Tannins, polyphenolic compounds of which GALALCOOL^{®} is only one example, are found at relatively high concentration in many edible plants. They are astringent and thus typically result in the precipitation of proteins, and furthermore, given that most viruses have a protein capsid (shell) interfacing with the external environment, this could be of central importance in removing viruses from free distribution in the mouth and throat (A Manual of Materia Medica and Pharmacology, p. 282. DMR Culbreck. Lea and Febiger, Philadelphia, 1927). In addition, this would suggest an ability, like that of zinc, to interfere with the attachment of certain viruses to cells of mucous membranes, while its very low water solubility suggests that it may preferentially sequester at different locations in the mouth, specifically those not easily wetted, unlike zinc salts. Also, it has been reported that polyphenols (such as the flavonoid compound quercetin) have antibacterial properties, hence possessing other potential benefits to oral health, and not just as a potential anti-viral agent (TPT Cushnie and AJ Lamb, Antimicrobial Activity of Flavonoids. International Journal of Antimicrobial Agents (2005) 26:343). However, as tannins typically are dark-colored compounds, only certain tannins will be considered as potential co-ingredients; tannins typically stain tooth enamel, so it is important that only colorless or low-color tannins be used in certain embodiments of the invention. They may also stain toothbrush bristles, producing another psychologically objectionable result. GALALCOOL^{®} or tara tannins will display potentially synergistic efficacy with the other component ingredients of the composition of the invention. The ingredients in the compositions of embodiments of the invention are at concentrations known not to be toxic, even if the product is inadvertently completely swallowed every time it is used. As for concerns on the toxicology of tannins, the Select Committee on GRAS substances has weighed in on the potential toxicity of tannic acid (as a hydrolysable gallotannin prototype) as follows (from https://www.fda.gov/Food/IngredientsPackagingLabeling/GRAS/SCOGS/ucm261485.htm, accessed Aug. 23, 2017): "The Select Committee, therefore, in the light of the foregoing concludes that:
"There is no evidence in the available information on tannic acid (hydrolyzable gallotannins) that demonstrates or suggests reasonable grounds to suspect a hazard to the public when it is used at levels that are now current and in the manner now practiced. However, it is not possible to determine, without additional data, whether a significant increase in consumption would constitute a dietary hazard."

Finally, another ingredient, zinc protoporphyrin IX (RF Labbe, et al., Zinc Protoporphyrin: A Metabolite with a Mission. Clinical Chemistry (1999) 45:2060-2072), may be a constituent of the orally administered product. In addition to being yet another zinc source, in this case of longer persistence, and an astringent in its own right, it is a known carrier of divalent ions (such as zinc ion), thus creating a more persistent zinc source that assists in conveying zinc to more extensive areas of the mouth and pharynx, and itself prolonging astringency in the latter areas. Zinc salts, being water soluble, will more easily wash out of the mouth and areas continuous with and beyond the oral cavity.

Because each ingredient has different physical and chemical properties, however, notwithstanding their commonality of astringency, a synergistic effect will result because of their differences in structural stability, affinities for each other, affinities for different surfaces in the mouth and pharynx, and tendencies to associate and adhere to different secretions.

Although currently available over-the-counter toothpastes address the need for the prevention of dental caries (bacteria-induced tooth decay leading to cavities), gingivitis, amelioration of sensitive teeth, and oral hygiene in general, the novel toothpastes described here may work in a synergistic mode by combining multiple ingredients. It can be expected that since the surface affinity of each ingredient differs according to specific tissue (e.g., gums vs. mucous membranes of the pharynx vs. tooth enamel), that the different ingredients will work to create a more thorough exposure of different tissues to the effective ingredients; furthermore, different strains of bacteria and viruses may differ substantially in susceptibility to the different ingredients, so that diversity in the ingredients should have more effect than their use at simply the highest safe concentration of each single ingredient known to be effective. The ingredients in the novel toothpaste will be present in a unique combination that includes ingredients that will enhance the ability of the toothpaste to prevent the acquisition of a number of conditions, in addition to those caused by coronavirus according to the present invention, including the common cold and a host of other pathological conditions caused by pathogenic viruses, bacteria, and fungi, or possibly other conditions (e.g., canker sores, ear infections, etc.). The consumer of the toothpaste should preferably need only brush his teeth once per day to attain desired results.

The following examples are presented in order to more fully illustrate the preferred embodiments of the invention. They should in no way be construed, however, as limiting the broad scope of the invention.

### EXAMPLES

### Example 1

Initially, quercetin was tried as a tannin component for a toothpaste and was added to a base toothpaste. At 20 µg of quercetin per 135 grams of base toothpaste, it was determined visually that more quercetin could not be added without risking the cumulative staining of a user's toothbrush and, eventually, that user's dental surface. A highly purified extract of gallic tannins (namely, GALALCOOL^{®}) that is relatively inexpensive and commercially available in bulk was then used. It was found that 500 mg of GALALCOOL^{®} can be added to a 135 gram tube of toothpaste without producing staining even after 6 months of daily use.

In another experiment, 6% aqueous suspensions of a highly purified extract of gallic tannins (GALALCOOL^{®}) and pure quercetin were prepared. One clear nylon bristle toothbrush was placed into each suspension and heated for 150 seconds (2.5 minutes) in an 1100W microwave oven. Separately an aqueous thick paste of a highly purified extract of gallic tannins (GALALCOOL^{®}) at 20× the loading of the quercetin suspension was also prepared. A separate toothbrush was heated similarly in that GALALCOOL^{®} paste. The toothbrushes were then rinsed in tap water. The two toothbrushes exposed to GALALCOOL^{®} showed no staining, while the toothbrush soaked in quercetin had a yellowish residual stain. In addition, the water from the cups with the 6% aqueous suspensions were decanted. The quercetin cup showed obvious sediment, while the GALALCOOL^{®} cup shows no such sediment.

### Example 2

The present inventors developed a toothpaste to combat the common cold by inactivating the pre-infection virus in the oral cavity. The approach is to deactivate virus on contact through use of a select tannin. Tannins are known to precipitate protein, and viruses are jacketed with protein. Therefore, tannin, especially if water-soluble and hydrolyzable, can cause disturbance to individual virions. In addition, it is sufficient to cause the virions to agglomerate (aggregate) into a mass too large to pass through membrane tissue where they would otherwise cause an active infection.

Using a mixture of a select tannin and a small amount of zinc gluconate added to a standard toothpaste base, when brushed daily onto the dental surface and foamed into the oral cavity a coating is formed that safely provides two modes of action: First, a barrier is set up to inhibit penetration of the cold virions through the oral membranes. Second, the tannin precipitates the protein of the virions causing them to agglomerate into an inactive state. The zinc salt provides zinc ions which can help adhere the tannins in the mouth. Zinc ions themselves can cross-link tannins to proteins, thus acting synergistically in agglomerating virions, and also can block proteins serving as ligands in binding the virions to cell receptors required for entry of the virions into their host cells.

### Coronavirus:

Taking the active ingredients (tannins and zinc ions) and adapting them safely into an inhalant form and dispersed into the lung can cause coronavirus virions (or other virions, such as influenza virions) to agglomerate, effectively inactivating the virus with minimal toxicological risk.

To illustrate interaction between the tannin molecules and the virion a substrate is coated with the active ingredients (tannins and zinc ions) and then coated with virions. The virus surfaces and their agglomeration are observed in micrographs produced under an electron microscope. An inhaler with these ingredients is tested on animals to evaluate toxicological or respiratory irritant issues, if any. Such an inhaler would be used perhaps once a day. Then, an animal model is used to show efficacy of these ingredients against lung-incubatable viruses.

This is a novel approach that is not a vaccination-based (i.e. strain-specific) approach. While it may be the case that the agglomeration of the virion can lead to be immune system being able to better identify the virus and cause it to create antibodies and an immune reaction on a viral-specific basis, even without the creation of antibodies and an immune response, the agglomeration of virions is a novel method of inactivating pre-infection virions independent of the virus. Consequently, one advantage of this approach over a vaccination-based approach is that it could inhibit other lung-incubating virions, including mutations of the new COVID-19 coronavirus strain.

## Claims

1. A composition formulated as a toothpaste for use in the prevention or inhibition of upper respiratory and pharyngeal infections, wherein the infection is a coronavirus infection, the composition comprising a dentifrice gel or paste comprising:
(1) a free zinc salt, wherein the free zinc salt ranges from 0.1 % to 0.5 % by weight of the composition; and
(2) a highly purified extract of gallic tannins or tara tannins that do not stain tooth enamel, and wherein the tannins are 0.01 % to 10 % by weight of the composition.

2. The composition for use according to claim 1, wherein the composition is stored in a malleable or squeezable tube.

3. The composition for use according to claim 1, wherein the coronavirus infection is COVID-19.

4. The composition for use according to claim 1, wherein the zinc salt is an organic zinc salt selected from zinc gluconate, zinc lactate, zinc acetate, or combinations thereof.

5. The composition for use according to claim 1, wherein the zinc salt is an inorganic zinc salt selected from zinc chloride, zinc sulphate, zinc carbonate, or combinations thereof.

6. The composition for use according to claim 1, further comprising a fluoride compound.

7. The composition for use according to claim 1, wherein the tara tannins are a highly purified extract from pods from *Caesalpinia spinosa.*

## Patentansprüche

1. Zusammensetzung, die als Zahnpasta formuliert ist, zur Verwendung bei der Vorbeugung oder Hemmung von Infektionen der oberen Atemwege und des Rachens, wobei die Infektion eine Coronavirus-Infektion ist, wobei die Zusammensetzung ein Zahnputzgel oder eine Zahnputzpaste umfasst, umfassend:
(1) ein freies Zinksalz, wobei das freie Zinksalz im Bereich von 0,1 bis 0,5 Gew.-% der Zusammensetzung liegt; und
(2) einen hoch gereinigten Extrakt aus Gallotanninen oder Tara-Tanninen, die den Zahnschmelz nicht verfärben, und wobei die Tannine 0,01 bis 10 Gew.-% der Zusammensetzung ausmachen.

2. Zusammensetzung zur Verwendung nach Anspruch 1, wobei die Zusammensetzung in einer verformbaren oder zusammendrückbaren Tube aufbewahrt wird.

3. Zusammensetzung zur Verwendung nach Anspruch 1, wobei die Coronavirus-Infektion COVID-19 ist.

4. Zusammensetzung zur Verwendung nach Anspruch 1, wobei das Zinksalz ein organisches Zinksalz ist, das aus Zinkgluconat, Zinklactat, Zinkacetat oder Kombinationen davon ausgewählt ist.

5. Zusammensetzung zur Verwendung nach Anspruch 1, wobei das Zinksalz ein anorganisches Zinksalz ist, das aus Zinkchlorid, Zinksulfat, Zinkcarbonat oder Kombinationen davon ausgewählt ist.

6. Zusammensetzung zur Verwendung nach Anspruch 1, die ferner eine Fluoridverbindung umfasst.

7. Zusammensetzung zur Verwendung nach Anspruch 1, wobei die Tara-Tannine ein hoch gereinigter Extrakt aus Schoten von *Caesalpinia spinosa* sind .

## Revendications

1. Composition formulée sous forme de pâte dentifrice pour une utilisation dans la prévention ou l'inhibition des infections des voies respiratoires supérieures et du pharynx, l'infection étant une infection à coronavirus, la composition comprenant un gel ou une pâte dentifrice comprenant :
(1) un sel de zinc libre, le sel de zinc libre représentant 0,1 % à 0,5 % en poids de la composition ; et
(2) un extrait hautement purifié de tanins galliques ou de tanins de tara qui ne tachent pas l'émail des dents, et les tanins représentant 0,01 % à 10 % en poids de la composition.

2. Composition pour une utilisation selon la revendication 1, la composition étant conservée dans un tube malléable ou compressible.

3. Composition pour une utilisation selon la revendication 1, dans laquelle l'infection à coronavirus est la COVID-19.

4. Composition pour une utilisation selon la revendication 1, dans laquelle le sel de zinc est un sel de zinc organique choisi parmi le gluconate de zinc, le lactate de zinc, l'acétate de zinc ou une de leurs combinaisons.

5. Composition pour une utilisation selon la revendication 1, dans laquelle le sel de zinc est un sel de zinc inorganique choisi parmi le chlorure de zinc, le sulfate de zinc, le carbonate de zinc ou une de leurs combinaisons.

6. Composition pour une utilisation selon la revendication 1, comprenant en outre un composé fluoré.

7. Composition pour une utilisation selon la revendication 1, dans laquelle les tanins de tara sont un extrait hautement purifié provenant des gousses de *Caesalpinia spinosa.*
